# EUROPÄISCHE PATENTSCHRIFT

(11) **EP 1 462 431 B1**
(45) Veröffentlichungstag und Bekanntmachung des Hinweises auf die Patenterteilung: **14.08.2013**
(21) Anmeldenummer: 04100260.1
(22) Anmeldetag: 26.01.2004
(51) Int. Cl.: C07C 29/80, C07C 31/12, B01D 61/02, B01D 61/36

(54) **Verfahren zur Abtrennung von 2-Butanol aus tert.-Butanol/Wasser-Gemischen**
Process for the separation of 2-butanol from tert.-butanol/water mixtures
Procédé pour la séparation de 2-butanol d' un mélange tert.-butanol/eau

(30) Priorität: 22.03.2003 DE 10312916
(43) Veröffentlichungstag der Anmeldung: 29.09.2004
(73) Patentinhaber: Evonik Oxeno GmbH, 45772 Marl (DE)
(72) Erfinder: Beckmann, Andreas, Dr., 45657 Recklinghausen (DE); Reusch, Dieter, Dr., 45772 Marl (DE); Kuppinger, Franz-Felix, Dr., 45768 Marl (DE)
(74) Vertreter: Hirsch, Hans-Ludwig

(56) Entgegenhaltungen:
- EP-A1- 0 304 770
- DATABASE CHEMABS [Online] CHEMICAL ABSTRACTS SERVICE, COLUMBUS, OHIO, US; MATSUMOTO, HIROYO ET AL: "Recovery of butanol from wastewaters" XP002279776 gefunden im STN Database accession no. 1988:173020 & JP 62 270537 A2 (MITSUBISHI HEAVY INDUSTRIES, LTD., JAPAN) 24. November 1987 (1987-11-24)
- DATABASE CHEMABS [Online] CHEMICAL ABSTRACTS SERVICE, COLUMBUS, OHIO, US; INASHIMA, MAKOTO ET AL: "Purification of a crude tert-butyl alcohol" XP002279771 gefunden im STN Database accession no. 1975:605762 & JP 500 163 39B B4 (MARUZEN OIL COMPANY, LTD., JAPAN) 12. Juni 1975 (1975-06-12)

## Beschreibung

Die vorliegende Erfindung betrifft ein Verfahren zur Abtrennung von 2-Butanol (im Text auch als sekundäres Butanol oder SBA bezeichnet) aus tert.-Butanol/Wasser-Gemischen, die bei der Spaltung von tert.-Butanol (TBA), insbesondere solchem hergestellt aus technischen C₄-Kohlenwasserstoffgemischen, zu Isobuten und Wasser anfallen.

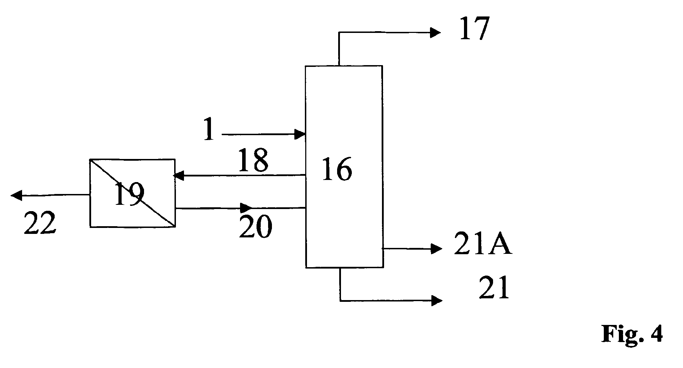

Eine Abtrennung von SBA aus einem technischen Gemisch, das SBA, TBA und Wasser aufweist, wobei der Massenanteil an Wasser in dem Gemisch sicher größer ist als die Grenzkonzentrationen der beiden Azeotrope TBA/Wasser und SBA/Wasser verbindenden Destillationsgrenzlinie, wobei in einem ersten Schritt aus dem Gemisch mit Hilfe eines Destillationsverfahrens Wasser abgetrennt wird und bei dem in einem zweiten Schritt das Gemisch destillativ in einem SBA aufweisenden Strom und einem überwiegend TBA und Wasser aufweisenden Strom getrennt wird, ist aus EP 0 304 770 A1 bekannt. Isoliert betrachtet, findet es in den in Figur 4 gezeigten Kolonnen 4 und 5 statt.

Bei dem Eingangsstrom 25 des in Figur 4 der EP 0 304 770 Al gezeigten Prozesses handelt es sich um ein Gemisch aus Wasser, TBA und SBA, was allerdings aufgrund seines dominanten SBA-Anteils nicht dem Oberbegriff des Anspruch 1 entspricht.

Isobuten ist ein Ausgangsstoff für die Herstellung von Butylkautschuk, Polyisobutylen, Isobuten-Oligomeren, verzweigten C₅-Aldehyden und C₅-Carbonsäuren. Weiterhin wird es als Alkylierungsmittel und als Zwischenprodukt zur Erzeugung von Peroxiden eingesetzt.

In technischen Strömen liegt Isobuten zusammen mit gesättigten und ungesättigten C₄-Kohlenwasserstoffen vor. Aus diesen Gemischen kann Isobuten wegen der geringen Siedepunktsdifferenz bzw. des sehr geringen Trennfaktors zwischen Isobuten und 1-Buten durch Destillation nicht wirtschaftlich abgetrennt werden. Daher wird Isobuten aus technischen Kohlenwasserstoffgemischen dadurch gewonnen, dass Isobuten zu einem Derivat umgesetzt wird, das sich leicht vom übrig gebliebenden Kohlenwasserstoffgemisch abtrennen lässt, und dass das isolierte Derivat zu Isobuten und Derivatisierungsmittel zurückspalbet wird.

Üblicherweise wird Isobuten aus C₄-Schnitten, beispielsweise der C₄-Fraktion eines Steamcrackers, wie folgt abgetrennt. Nach Entfernung des größten Teils der mehrfach ungesättigten Kohlenwasserstoffe, hauptsächlich Butadien, durch Extraktion(-sdestillation) oder Selektivhydrierung zu linearen Butenen wird das verbleibende Gemisch (Raffinat I oder hydriertes Crack-C₄) mit Alkohol oder Wasser umgesetzt. Bei der Verwendung von Methanol als Alkohol entsteht Methyl-tert.-butylether (MTBE) und bei Einsatz von Wasser tert.-Butanol (TBA). Nach ihrer Abtrennung können beide Produkte in Umkehrung ihrer Bildung zu Isobuten gespalten werden.

Die Spaltung von TBA lässt sich leichter als die Spaltung von MTBE durchführen und ergibt weniger Nebenprodukte und ist somit das bevorzugte Verfahren zur Gewinnung von Isobuten. Beyorzugt wird die Spaltung von TBA in Gegenwart einer Säure in der Gas- oder Flüssigphase unter Teilumsatz von TBA durchgeführt.

Werden zur Herstellung von TBA aus Isobuten Isobuten-haltige Kohlenwasserstoffströme, die auch lineare Butene enthalten, eingesetzt, entsteht in geringen Mengen auch 2-Butanol (SBA).

Dies ist solange nicht weiter kritisch, wie das entstandene Reaktionsgemisch in ein reines TBA oder in ein TBA/Wasser-Azeotrop aufgearbeitet wird. Dabei wird wegen des nur geringen 2-Butanolgehalts im Reaktionsgemisch die maximal zulässige 2-Butanolkonzenzentration von beispielsweise 0,2 Massen-% im TBA oder im TBA/Wasser-Azeotrop nicht überschritten.

Wird das technische TBA oder TBA/Wasser-Azeotrop allerdings unter Teilumsatz in Isobuten und Wasser gespalten, so fällt nach Abtrennung des entstandenen Isobutens ein TBA/Wasser-Gemisch an, in dem das 2-Butanol (SBA) angereichert ist. Dieses Gemisch ist ohne eine Abtrennung von 2-Butanol nicht zur Herstellung von marktgängigen TBA oder TBA/Wasser-Azeotrop geeignet. Ebenfalls ist es nicht zweckmäßig, aus diesem Gemisch Isobuten herzustellen, weil mit zunehmenden 2-Butanolgehalt auch die Konzentration von linearen Butenen im Isobuten steigt, wodurch dessen Spezifikation nicht eingehalten werden kann. Daher ist es notwendig, einen Teil des 2-Butanols unter Vermeidung von TBA-Verlusten auszuschleusen.

Aufgabe der vorliegenden Erfindung war es deshalb ein Verfahren bereitzustellen, mit welchem es möglich ist, SBA aus Gemischen, die SBA, TBA und Wasser aufweisen, abzutrennen, ohne dass es zu Verlusten an TBA kommt.

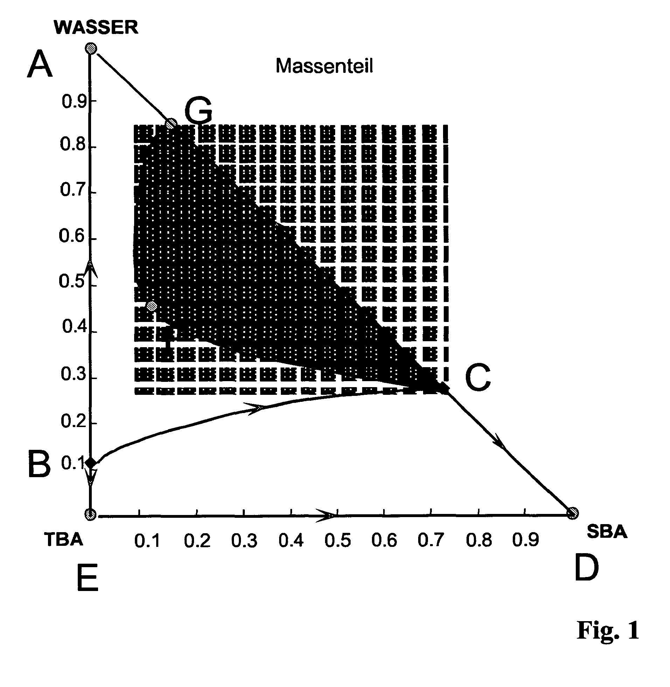

Das Dreistoffgemisch aus SBA, TBA und Wasser ist allerdings destillativ schwierig zu trennen, da in diesem Dreistoffsystem eine Destillationsgrenzlinie (in der Literatur manchmal auch als Grenzdestillationslinie bezeichnet) verläuft, die das binäre Azeotrop Wasser/TBA bei ca. 11 Massen-% Wasser (in der Literatur sind Werte bei Normaldruck von 10 bis 12,5 Massen-% zu finden) (Punkt B in Fig. 1) mit dem binären Azeotrop Wasser/SBA bei ca. 28 Massen-% Wasser (in der Literatur sind Werte bei Normaldruck von 26,7 bis 32 Massen-% zu finden) (Punkt C in Fig. 1) verbindet. Durch diese Destillationsgrenzlinie werden zwei Destillationsfelder getrennt. Kennzeichnend für das obige Dreistoffsystem, dargestellt in Figur 1, sind zwei Destillationsfelder: Destillationsfeld 1 im Bereich A-B-C-A und Destillationsfeld 2 im Bereich B-E-D-C-B. Im Destillationsfeld 1 findet man als Schwersieder Wasser, der Leichtsieder in diesem Bereich ist das TBA/Wasser-Azeotrop und der Mittelsieder das SBA/Wasser-Azeotrop, das nicht in reiner Form abgetrennt werden kann.

Um SBA aus einem TBA-Isobuten-Anlagenverbund auszuschleusen, ist es am wirtschaftlichsten, den SBA-reichsten Strom dazu zu verwenden. Die bei der TBA-Spaltung erhaltenen Ströme weisen aber einen relativ geringen Gehalt an SBA auf. Üblicherweise weisen sie Zusammensetzungen auf, die im Destillationsfeld 1 liegen. Meistens enthalten diese Ströme auch noch geringe Mengen weiterer Stoffe, deren Gegenwart man in diesem Zusammenhang allerdings nicht zu betrachten braucht. Versucht man ein solches Gemisch mit einer Zusammensetzung im Bereich des Destillationsfeldes 1 destillativ aufzuarbeiten, kann man entweder reines Wasser als Schwersieder und ein Gemisch aus SBA/TBA/Wasser als Kopffraktion gewinnen oder aber im Destillat einer Kolonne als leichtestsiedendes Gemisch das TBA/Wasser-Azeotrop und im Sumpf eine höher siedende Mischung aus SBA/TBA/Wasser mit einem hohen Wasseranteil erhalten. Man kann also aus Massenbilanzgründen und aufgrund des ungünstigen Verlaufs der Destillationslinien in keiner Weise den Gehalt an SBA derart erhöhen, dass eine Ausschleusung dieses Stroms wirtschaftlich sinnvoll ist. Auch die im System vorhandene Mischungslücke (siehe Figur 1: C-F-G-C) kann nicht wirtschaftlich für die Trennung der Komponenten oder deren Anreicherung verwendet werden.

Überraschenderweise wurde aber gefunden, dass aus einem Produktionsstrom, der Wasser, SBA und TBA aufweist und dessen Zusammensetzung im Bereich des Destillationsfelds 1 liegt, insbesondere aus einem Produktionsstrom, in dem SBA angereichert ist, SBA praktisch ohne Verluste an TBA abgetrennt werden kann, wenn aus dem als Einsatzgemisch verwendeten Produktionsstrom mit Hilfe einer Membran so viel Wasser abgetrennt wird, dass das Retentat eine Zusammensetzung aufweist, die im Bereich des Destillationsfelds 2 liegt und somit das Retentat destillativ in SBA und in ein TBA/Wasser-Gemisch aufgetrennt werden kann.

Gegenstand der Erfindung ist demnach ein Verfahren zur Abtrennung von SBA aus einem technischen Gemisch, das SBA, TBA und Wasser aufweist, wobei der Massenanteil an Wasser in diesem Gemisch größer ist als die Grenzkonzentrationen der die beiden Azeotrope TBA/Wasser und SBA/Wasser verbindenden Destillationsgrenzlinie, also das Gemisch hinsichtlich seiner SBA/TBA/Wasser-Zusammensetzung im Bereich des Destillationsfelds 1 liegt, welches dadurch gekennzeichnet ist, dass aus dem Gemisch mit Hilfe einer Membran so viel Wasser abgetrennt wird, dass das Retentat hinsichtlich seiner SBA/TBA/Wasser-Zusammensetzung einen Massenanteil an Wasser aufweist, der kleiner ist als die Grenzkonzentration der die beiden Azeotrope TBA/Wasser und SBA/Wasser verbindenden Destillationsgrenzlinie also hinsichtlich seiner SBA/TBA/Wasser-Zusammensetzung im Bereich des Destillationsfeld 2 liegt, und dass das Retentat destillativ in einen SBA aufweisenden Strom und einen überwiegend TBA und Wasser aufweisenden Strom getrennt wird.

Im erfindungsgemäßen Verfahren erfolgt also die SBA-Abtrennung durch eine Kombination von Wasserabtrennung mit Hilfe einer Membran und Destillation des an Wasser verarmten Retentats.

Durch das erfindungsgemäße Verfahren ist es nun möglich SBA aus Gemischen abzutrennen, die SBA, TBA und Wasser aufweisen, wobei der Massenanteil an Wasser in diesen Gemischen größer ist als die Grenzkonzentrationen der die beiden Azeotrope TBA/Wasser und SBA/Wasser verbindenden Destillationsgrenzlinie, und damit rein destillativ nicht trennbar sind. Durch die Verwendung des Membranverfahrens kann auf die Verwendung von Schleppmitteln oder anderen Fremdstoffen verzichtet werden, so dass auf eine aufwändige Abtrennung dieser Hilfsstoffe vermieden werden kann und die Gefahr der Verunreinigung der Produkte durch diese Hilfsstoffe bei der Aufarbeitung ausgeschlossen wird.

Das erfindungsgemäße Verfahren wird nachfolgend beschrieben, ohne dass das Verfahren auf diese Ausführungsformen beschränkt sein soll.

Das erfindungsgemäße Verfahren zur Abtrennung von SBA aus einem technischen Gemisch, das SBA, TBA und Wasser aufweist, wobei der Massenanteil an Wasser in diesem Gemisch größer ist als die Grenzkonzentrationen der die beiden Azeotrope TBA/Wasser und SBA/Wasser verbindenden Destillationsgrenzlinie, also das Gemisch hinsichtlich seiner SBA/TBA/Wasser-Zusammensetzung im Bereich des Destillationsfelds 1 liegt, zeichnet sich dadurch aus, dass aus dem Gemisch mit Hilfe einer Membran so viel Wasser abgetrennt wird, dass das Retentat hinsichtlich seiner SBA/TBA/Wasser-Zusammensetzung einen Massenanteil an Wasser aufweist, der kleiner ist als die Grenzkonzentration der die beiden Azeotrope TBA/Wasser und SBA/Wasser verbindenden Destillationsgrenzlinie, also hinsichtlich seiner SBA/TBA/Wasser-Zusammensetzung im Bereich des Destillationsfeld 2 liegt, und dass das Retentat destillativ in einen SBA aufweisenden Strom und einen TBA und Wasser aufweisenden Strom getrennt wird.

Die Abtrennung des Wassers aus dem SBA-haltigen Einsatzgemisch mit Hilfe einer Membran kann durch Umkehr-Osmose (Retentat und Permeat sind flüssig), durch Pervaporation (flüssiges Retentat, dampfförmiges Permeat) oder durch Dampf-Permeation (Retentat und Permeat dampfförmig) erfolgen. Weiterhin ist eine Abtrennung durch gleichzeitige Pervaporation und Dampfpermeation möglich. Bevorzugt erfolgt die erfindungsgemäße Abtrennung des Wassers mit Hilfe einer Membran durch Pervaporation (flüssiges Retenat, dampfförmiges Permeat)

Zur Wasserabtrennung durch Umkehr-Osmose, Pervaporation oder Dampf-Permeation können handelsübliche hydrophile Membrane verwendet werden. Diese Membrane können Polymermembrane oder anorganische Membrane sein.

Im erfindungsgemäßen Verfahren können beispielsweise Polymermembrane der Firmen Sulzer Chemtech, CM-Celfa, GKSS oder Sophisticated Systems (Polyimidmembran) eingesetzt werden. Verwendbare Typen sind z.B. Pervap 2200, Pervap 2201, Pervap 2202 oder Pervap 2510 von Sulzer oder Typ 2S-DP-H018 von Sophisticated Systems. Als anorganische Membrane können beispielsweise die nachfolgend aufgeführten verwendet werden: SMS (Sulzer Chemtech); Silica (Pervatech); NaA (Mitsui oder Smart Chemical). Weiterhin können auch Kombinationen aus anorganischer Membran bzw. anorganischem Trägermaterial und einer Polymermembran bzw. aufgebrachten Polymertrennschicht eingesetzt werden.
Anorganische Membranen bieten unter Umständen Vorteile, da sie oft bei höheren Temperaturen stabil sind und sich auch mit überhitztem Dampf besser betreiben lassen. Ihre Stabilität gegenüber organischen Komponenten kann in manchen Fällen ebenfalls höher sein. Polymermembrane bieten dagegen den Vorteil, dass sie keine katalytischen Eigenschaften, wie z.B. für Polymerisationsreaktionen aufweisen, wie dies bei anorganischen Membranen der Fall sein kann.
Die erfindungsgemäße Wasserabtrennung erfolgt an den anorganischen Membranen bevorzugt bei einer Temperatur von 20 bis 200 °C und an den Polymermembranen bevorzugt bei einer Temperatur von 20 bis 150 °C. Besonders bevorzugt wird die Wasserabtrennung an beiden Membrantypen bei einer Temperatur von 60 bis 140 °C durchgeführt.

Das erfindungsgemäße Verfahren wird vorzugsweise bei einem Druck des der Membraneinheit zugeführten Gemisches (Flüssig, dampfförmig oder als Mischphase) von 0,5 bis 30 bar, bevorzugt von 0,8 bis 20 bar durchgeführt. Der Druck auf der Permeatseite der Membran beträgt vorzugsweise von 0,001 bis 1 bar.

Bei Polymermembranen beträgt der Differenzdruck vorzugsweise von 0,01 bis 20 bar und bei anorganischen Membranen vorzugsweise von 0,01 bis 30 bar. Besonders bevorzugt liegen die Differenzdrücke im Bereich von 1 bis 5 bar. Der Massenstrom (kg Permeat je Quadratmeter Membranoberfläche je Stunde) beträgt vorzugsweise von 0,1 bis 10 kg/(m²h), besonders bevorzugt von 1 bis 8 kg/(m²h). Das als Permeat abgetrennte Wasser weist vorzugsweise einen Gehalt an organischen Inhaltsstoffen von kleiner 10 Massen-%, bevorzugt kleiner 5 Massen-% und ganz besonders bevorzugt von 3 bis 0,05 Massen-%, insbesondere an TBA auf. Es können auch kleinere Werte bis zu 0,001 Massen-% erreicht werden, wobei dies meistens nicht erforderlich und nicht wirtschaftlich sinnvoll ist.

Aus dem wässrigen Permeat können TBA und SBA zusammen aufkonzentriert und von reinem bzw. nahezu reinem Wasser abgetrennt werden. Diese Abtrennung kann z.B. rein destillativ erfolgen, da ein Gemisch mit einer Zusammensetzung im Bereich des Destillationsfeldes 1 vorliegt und durch Destillation reines bzw. nahezu reines Wasser als Schwersieder und ein Gemisch aus SBA/TBA/Wasser als Kopffraktion gewonnen werden kann. Das Gemisch aus der Kopffraktion kann ganz oder nach Ausschleusung eines Teilstroms partiell in das Membranmodul zur erneuten Abtrennung von Wasser zurückgeführt werden.

Optional kann das Permeat ganz oder teilweise direkt verwendet werden, beispielsweise als Prozesswasser in einer Anlage zur TBA-Synthese.

Das Retentat liegt hinsichtlich seines Wassergehaltes im Destillationsfeld 2, weist also einen Massenanteil an Wasser auf kleiner als der Wassergehalt eines Gemisches mit einer Zusammensetzung entsprechend der die Azeotrope SBA/Wasser und TBA/Wasser verbindenden Destillationsgrenzlinie B-C. Vorzugsweise beträgt der Wassergehalt bezogen auf das Dreikomponentensystem SBA/TBA/Wasser bei SBA-Gehalten von 0,0001 bis 6 Massen-% weniger als 11 Massen-%, bevorzugt weniger als 10 Massen-% und besonders bevorzugt weniger als 9,5 Massen-%. Bei SBA-Gehalten von 6,01 bis 15 Massen-% beträgt der Wassergehalt bezogen auf das Dreikomponentensystem SBA/TBA/Wasser bevorzugt weniger als 15 Massen-%, bevorzugt weniger als 14 und besonders bevorzugt weniger als 13 Massen-%. Weiterhin kann das Retentat bis zu 5 Massen-%, vorzugsweise bis zu 3 Massen-%, ganz besonders bevorzugt von 2,5 bis 0,01 Massen-% an weiteren Stoffen, beispielsweise C₈-Olefinen oder -Alkoholen, aufweisen.

Die destillative Auftrennung von bei dem erfindungsgemäßen Verfahren anfallenden Stoffströmen, insbesondere des Retentats, kann in ein oder mehreren Kolonnen mit Einbauten, die aus Böden, rotierenden Einbauten, ungeordneten und/oder geordneten Packungen bestehen, durchgeführt werden. Vorzugsweise erfolgt die destillative Trennung in einer einzigen Kolonne.

Bei den Kolonnenböden können folgende Typen zum Einsatz kommen:
Böden mit Bohrungen oder Schlitzen in der Bodenplatte.
Böden mit Hälsen oder Kaminen, die von Glocken, Kappen oder Hauben überdeckt sind.
Böden mit Bohrungen in der Bodenplatte, die von beweglichen Ventilen überdeckt sind.
Böden mit Sonderkonstruktionen.

In Kolonnen mit rotierenden Einbauten wird der Rücklauf entweder durch rotierende Trichter versprüht oder mit Hilfe eines Rotors als Film auf einer beheizten Rohrwand ausgebreitet.

In dem erfindungsgemäßen Verfahren verwendete Kolonnen können regellose Schüttungen mit verschiedenen Füllkörpern eingesetzt werden. Sie können aus fast allen Werkstoffen - Stahl, Edelstahl, Kupfer, Kohlenstoff, Steingut, Porzellan, Glas, Kunststoffen usw. - und in verschiedenen Formen - Kugeln, Ringen mit glatten oder profilierten Oberflächen, Ringen mit Innenstegen oder Wanddurchbrüchen, Drahtnetzringen, Sattelkörper und Spiralen - bestehen. Packungen mit regelmäßiger Geometrie können z.B. aus Blechen oder Geweben aus Metall oder Kunststoff bestehen. Beispiele solcher Packungen sind Sulzer Gewebepackungen BX, Sulzer Lamellenpackungen Mellapak aus Metallblech, Hochleistungspackungen wie MellapakPlus, Strukturpackungen von Sulzer (Optiflow), Montz (BSH) und Kühni (Rombopak).

Die zur Trennung des aus dem Membranmodul erhaltenen Retentats eingesetzte Kolonne weist vorzugsweise eine theoretische Trennstufenzahl von 5 bis 70, bevorzugt eine theoretische Trennstufenzahl von 10 bis 60 auf. Der Zulaufboden hängt von der Zusammensetzung des Retentats ab. Es hat sich als vorteilhaft erwiesen, wenn die Einspeisung des Retentats auf den, von oben gezählten, 2. bis 55. theoretischen Boden, insbesondere auf den 3. bis 35. erfolgt.

Der Betriebsdruck der Kolonne zur Auftrennung des Retentats beträgt vorzugsweise von 0,1 bis 15 bar_{abs.} (bara), besonders bevorzugt von 0,8 bis 10 bara.

Bei der Destillation des Retentats fällt ein Sumpfprodukt als Strom an, das 2-Butanol und gegebenenfalls Hochsieder enthält. Der Gehalt an TBA darin beträgt vorzugsweise kleiner 1 Massen-%, bevorzugt kleiner 0,5 Massen-%. Als Kopfprodukt wird ein Gemisch aus TBA, Wasser und gegebenenfalls Leichtsieder abgezogen. Der Gehalt an 2-Butanol im Kopfprodukt beträgt vorzugsweise weniger als 4 Massen-%, insbesondere weniger als 3 Massen-%. Im Sumpf der Kolonne lässt sich 2-Butanol ohne oder nahezu ohne Hochsieder gewinnen, indem das 2-Butanol aus der Dampfphase des Verdampfers oder dampfförmig oder flüssig als Seitenstrom im Abtriebsteil der Kolonne abgezogen wird.

Die mit dem erfindungsgemäßen Verfahren erhaltenen aus dem Gemisch abgetrennten TBA-Fraktionen können für die bekannten Zwecke verwandt werden. Beispielsweise können sie als Ausgangsmaterial für die Herstellung von Isobuten dienen. Gegebenenfalls darin enthaltene Leichtsieder können destillativ abgetrennt werden.

Das abgetrennte 2-Butanol kann für die üblichen technischen Anwendungen genutzt werden. So kann es z.B. als Vorstufe für Methyl-ethylketon, als Lösemittel für Lacke und Harze, als Bestandteil von Bremsflüssigkeiten sowie als Bestandteil von Reinigungsmitteln eingesetzt werden. Darüber hinaus fmdet es Verwendung bei der Herstellung von Duftstoffen, Farbstoffen und Benetzungsmitteln.

Mit dem erfindungsgemäßen Verfahren kann 2-Butanol ohne Verluste von TBA aus beliebigen ternären Gemischen aus TBA, SBA und Wasser, die im Destillationsfeld 1 liegen, abgetrennt werden. Dies gelingt selbst dann noch, wenn die Gemische zusätzlich bis zu 5 Massen-% Hochsieder (wie z.B. aus Oligomerisierung von Isobuten hervorgegangene C₈- oder C₁₂-Kohlenwasserstoffe, C₈-Alkohole) und/oder Leichtsieder (wie z.B. Isobuten oder andere C₄-Kohlenwasserstoffe) aufweisen. Mittels des erfindungsgemäßen Verfahrens kann also 2-Butanol, insbesondere 2-Butanol, welches einen Gehalt an tert.-Butanol von vorzugsweise weniger als 1, bevorzugt weniger als 0,5 Massen-% aufweist, hergestellt werden.

Im erfindungsgemäßen Verfahren werden insbesondere TBA-Ströme, in denen 2-Butanol angereichert ist, aus Anlagen, in denen Isobuten aus TBA durch Wasserabspaltung hergestellt wird, eingesetzt. Diese Ströme enthalten meistens C₄-Kohlenwasserstoffe und Folgeprodukte von C₄-Olefinen als weitere Komponenten.

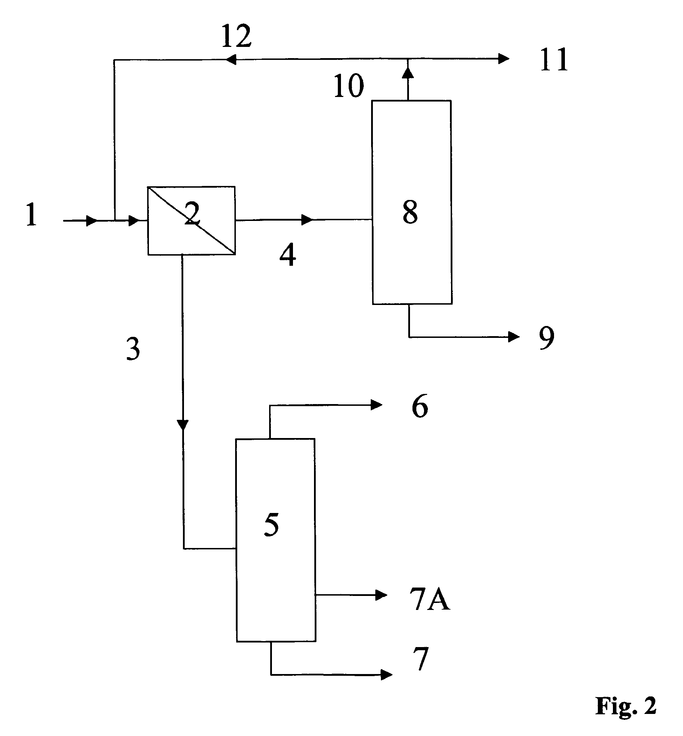

Das Blockschema einer Anlage, in der das erfindungsgemäße Verfahren durchgeführt werden kann, zeigt Fig. 2. Das Einsatzstoffgemisch (1) wird mit einem optionalen Teil (12) des Destillats (10) aus Kolonne (8) in die Membraneinheit (2) eingeleitet. Das an Wasser verarmte Retentat (3) wird in der Kolonne (5) in ein Kopfprodukt (6), das TBA, Wasser, geringe Mengen an SBA und gegebenenfalls Leichtsieder enthält, und in ein Sumpfprodukt (7) mit dem abzutrennenden 2-Butanol getrennt. Um 2-Butanol mit einem geringen Anteil an Hochsiedern zu gewinnen, kann dieses Produkt aus der Dampfphase des Verdampfers oder dampfförmig oder flüssig als Seitenstrom (7A) im Abtriebsteil der Kolonne (5) abgezogen werden. Das wässrige Permeat (4) wird in der Kolonne (8) in einen Wasserstrom (9) und in ein Kopfprodukt (10), das TBA, SBA, Wasser und gegebenenfalls Leichtsieder enthält, aufgetrennt. Dieses Kopfprodukt kann ganz oder nach Abtrennung einer Teilmenge (11) partiell in die Membraneinheit (2) zurückgeführt werden. Alternativ kann der Permeatstrom (4) direkt als Prozesswasser im Anlagenteil einer TBA-Synthese wieder eingesetzt werden. In diesem Falle wird die Kolonne (8) nicht benötigt und entfällt.

In einer anderen Ausführungsform des erfindungsgemäßen Verfahrens wird dem technischen Gemisch vor der Zuführung zur Membraneinheit destillativ Wasser entzogen. Durch Destillation des technischen Gemisches wird dieses in ein wasserreiches Sumpfprodukt und ein Kopfprodukt aufgetrennt, welches einen Wassergehalt aufweist, der zwar größer ist als die Grenzkonzentration der die beiden Azeotrope TBA/Wasser und SBA/Wasser verbindenden Destillationsgrenzlinie, also hinsichtlich seiner SBA/TBA/Wasser-Zusammensetzung im Bereich des Destillationsfelds 1 liegt, aber geringer als der ursprüngliche Gehalt an Wasser ist. Das so vorbehandelte technische Gemisch wird dann in einer Membraneinheit weiter an Wasser verarmt und entsprechend aufgearbeitet.

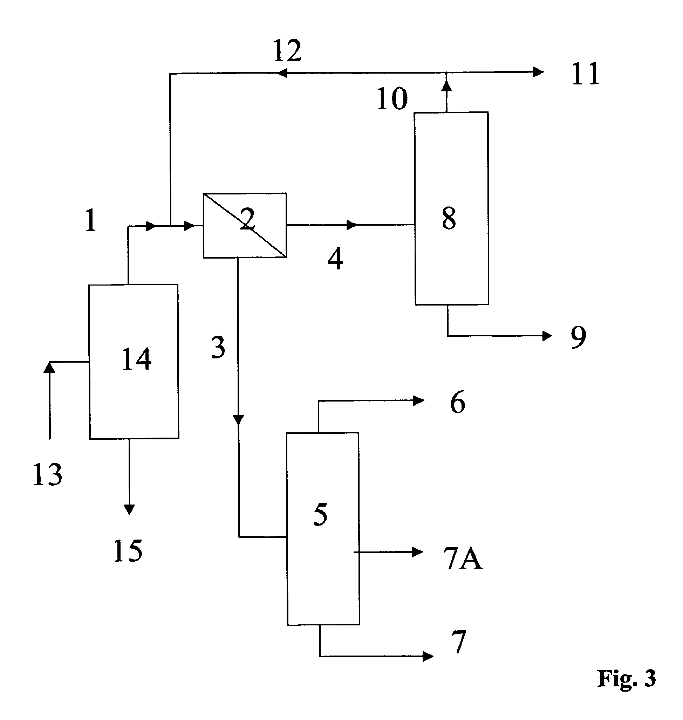

Ein Blockschema einer Variante der Anlage, in der diese Ausführungsart des erfindungsgemäßen Verfahrens durchgeführt werden kann, zeigt Fig 3. Das Einsatzstoffgemisch (13) wird zunächst in einer Kolonne (14) derart aufgearbeitet, dass im Sumpf der Kolonne ein wasserreicher Strom (15) ausgeschleust wird. Der auf diese Weise vorentwässerte Destillatstrom (1) wird nun mit einem optionalen Teil (12) des Destillats (10) aus Kolonne (8) in die Membraneinheit (2) eingeleitet. Das an Wasser verarmte Retentat (3) wird in der Kolonne (5) in ein Kopfprodukt (6), das TBA, Wasser und gegebenenfalls Leichtsieder enthält, und in ein Sumpfprodukt (7) mit dem abzutrennenden 2-Butanol getrennt. Auch in diesem Fall kann, um 2-Butanol mit einem geringen Anteil an Hochsiedern gewinnen zu können, das Produkt aus der Dampfphase des Verdampfers oder dampfförmig oder flüssig als Seitenstrom (7A) im Abtriebsteil der Kolonne (5) abgezogen werden. Das wässrige Permeat (4) wird in der Kolonne (8) in einen Wasserstrom (9) und in ein Kopfprodukt (10), das TBA, SBA, Wasser und gegebenenfalls Leichtsieder enthält. Dieses Kopfprodukt kann ganz oder nach Abtrennung einer Teilmenge (11) partiell in die Membraneinheit (2) zurückgeführt werden. Alternativ kann der Permeatstrom (4) direkt als Prozesswasser im Anlagenteil einer TBA-Synthese wieder eingesetzt werden. In diesem Falle wird die Kolonne (8) nicht benötigt und entfällt.

Der Vorteil dieser Variante liegt darin, dass in der Vorentwässerungskolonne (14) der Zulauf (1) zur Membraneinheit wasserärmer wird und somit im Destillationsfeld 1 näher an die Destillationsgrenzlinie BC rückt. Dies bedeutet, dass weniger Wasser aus dem Zulaufstrom (1) in der Membraneinheit abgetrennt werden muss, um in das Destillationsfeld 2 zu gelangen und dass das Membranmodul somit kleiner und günstiger gebaut werden kann.
Als Vorentwässerungskolonne kann eine üblicherweise zur Destillation verwendete Kolonne eingesetzt werden. Diese Kolonne weist vorzugsweise eine theoretische Trennstufenzahl von 5 bis 70, bevorzugt eine theoretische Trennstufenzahl von 10 bis 60 auf. Der Zulaufboden hängt von der Zusammensetzung des Zulaufs ab. Es hat sich als vorteilhaft erwiesen, wenn die Einspeisung des Zulaufs auf den, von oben gezählten, 2. bis 55. theoretischen Boden, insbesondere auf den 3. bis 35. erfolgt. Der Betriebsdruck der Vorentwässerungskolonne beträgt vorzugsweise von 0,1 bis 15 bar_{abs} (bara), besonders bevorzugt von 0,8 bis 10 bara.

In einer weiteren Ausführungsform des erfindungsgemäßen Verfahrens wird das zu trennende Gemisch als Zulauf in eine Kolonne geführt, in welcher im Seitenstrom mit Hilfe eines Membranmoduls Wasser ausgeschleust und das zurückgehaltene, an Wasser abgereicherte Gemisch wieder in die Kolonne zurückgeführt wird. Durch Destillation dieses Gemisches im Verstärkungsteil der Kolonne wird dieses in ein TBA-angereichertes Kopfprodukt und ein wasserreicheres Produkt in der Mitte der Kolonne aufgetrennt, welches einen Wassergehalt aufweist, der größer ist als die Grenzkonzentration der die beiden Azeotrope TBA/Wasser und SBA/Wasser verbindenden Destillationsgrenzlinie, also hinsichtlich seiner SBA/TBA/Wasser-Zusammensetzung im Bereich des Destillationsfeld 1 liegt. Dieses wasserreiche Gemisch wird als Seitenstrom aus der Destillationskolonne unterhalb des Zulaufs dampfförmig oder flüssig entnommen und in ein Membranmodul überführt. In diesem wird das wasserreiche Produkt in erfindungsgemäßer Weise durch Ausschleusen von Wasser als Permeat aufkonzentriert auf einen Gehalt an Wasser, kleiner als die Grenzkonzentration der die beiden Azeotrope TBA/Wasser und SBA/Wasser verbindenden Destillationsgrenzlinie. Dieses an Wasser abgereicherte Retentat wird durch Einspeisen an gleicher oder tiefere Stelle (in Bezug auf die Position der Entnahme des Seitenstroms) in den unteren Teil der Kolonne zurückgeführt, wodurch erreicht wird, dass das Gemisch im unteren Teil der Kolonne eine SBA/TBA/Wasser-Zusammensetzung mit einem Massenanteil an Wasser aufweist, der kleiner ist als die Grenzkonzentration der die beiden Azeotrope TBA/Wasser und SBA/Wasser verbindenden Destillationsgrenzlinie, und im Sumpf der Kolonne angereichertes SBA abtrennt werden kann.

Ein Blockschema einer Variante der Anlage, in der diese Ausführungsart des erfindungsgemäßen Verfahrens durchgeführt werden kann, zeigt Fig 4. Das Einsatzstoffgemisch (1) wird in den oberen Teil einer Kolonne (16) geleitet und derart aufgearbeitet, dass im Kopf der Kolonne ein TBA-reicher Strom (17) gewonnen wird. Unterhalb des Zulaufs wird ein dampfförmiger oder flüssiger Seitenstrom (18) entnommen und in die Membraneinheit (19) eingeleitet. Das an Wasser verarmte Retentat (20) wird wieder an gleicher oder tieferer Stelle (Zählweise der theoretischen Stufenzahl von oben nach unten) in die Kolonne (16) zurückgeführt. In dem Teil unterhalb dieser Rückführung befmdet man sich im Destillationsfeld 2 und kann ein Sumpfprodukt (21) mit dem abzutrennenden 2-Butanol gewinnen. Auch in diesem Fall kann, um 2-Butanol mit einem geringen Anteil an Hochsiedern gewinnen zu können, das Produkt aus der Dampfphase des Verdampfers oder dampfförmig oder flüssig als Seitenstrom (21A) im Abtriebsteil der Kolonne (16) abgezogen werden. Das wässrige Permeat (22) wird kontinuierlich aus dem Membranmodul ausgeschleust, wobei soviel Wasser ausgeschleust wird, dass die Zusammensetzung des erhaltenen Retentats im Destillationsfeld 2 liegt. Das Wasser kann als Prozesswasser im Anlagenteil einer TBA-Synthese wieder eingesetzt werden. Alternativ kann das Permeat (22) in einer nachgeschalteten Destillation so aufgearbeitet werden, dass reines Wasser als Sumpfprodukt erhalten wird und ein Kopfstrom gewonnen wird, der überwiegend TBA, andere organische Komponenten und geringe Mengen an Wasser aufweist.

Der Vorteil dieser Variante liegt darin, dass nur eine Kolonne erforderlich ist und dass mit dem Membranmodul keine vollständige Entwässerung des Stroms (18) erforderlich ist, sondern nur soviel Wasser ausgeschleust werden muss, um vom Destillationsfeld 1 nach 2 zu wechseln. Dadurch kann die benötigte Membranfläche kleiner ausfallen und günstiger gebaut werden.

Zur destillativen Auftrennung können hierfür technische Kolonnen, deren mögliche Bauarten bereits auf den vorangegangenen Seiten, insbesondere bei der Beschreibung der destillative Auftrennung von bei dem erfindungsgemäßen Verfahren anfallenden Stoffströmen beschrieben wurden, eingesetzt werden. Die Kolonne weist vorzugsweise eine theoretische Trennstufenzahl von 5 bis 120, bevorzugt eine theoretische Trennstufenzahl von 10 bis 90 auf. Der Zulaufboden hängt von der Zusammensetzung des Zulaufs ab. Es hat sich als vorteilhaft erwiesen, wenn die Einspeisung des Zulaufs auf den, von oben gezählten, 2. bis 85. theoretischen Boden, insbesondere auf den 3. bis 75. erfolgt. Der dampfförmige oder flüssige Seitenstrom wird unterhalb des Zulaufs auf der, von oben gezählten, 3. bis 110. theoretischen Stufe, bevorzugt auf der 4. bis 95. theoretischen Stufe entnommen und der an Wasser abgereicherte Strom (20) auf der 3. bis 111. theoretischen Stufe, bevorzugt auf der 5. bis 96. theoretischen Stufe wieder in die Kolonne (16) zurückgeführt. Der Betriebsdruck der Kolonne bei dieser Ausführungsart des erfindungsgemäßen Verfahrens beträgt vorzugsweise von 0,1 bis 15 bar_{abs}. (bara), besonders bevorzugt von 0,8 bis 10 bara.

Gewöhnliche Bauteile wie Pumpen, Verdichter, Ventile, Wärmetauscher und Verdampfer sind in den Blockschaltbildern nicht dargestellt, jedoch selbstverständliche Bauteile einer Anlage.

Die folgenden Beispiele sollen die Erfindung erläutern, ohne die Anwendungsbreite einzuschränken, die sich aus der Beschreibung und den Patentansprüchen ergibt.

### 1. Beispiel

Die Abtrennung von SBA erfolgte in einer nach Figur 2 realisierten Anlage, mit der Besonderheit, dass Kolonne (8), und damit die Ströme 9, 10, 11 und 12 entfielen. Der Kolonnendurchmesser der Kolonne (5) betrug dabei 50 mm. Es wurde eine Metall-Destillations-Packung mit 20 theoretischen Stufen installiert, der Zulauf erfolgte auf der, von oben gezählten, 5. theoretischen Stufe. Der Zulauf (1), der für die Versuche verwendet wurde, wurde aus einer großtechnischen Anlage zur Herstellung von Isobuten entnommen und wies die in Tabelle 1 angegebene Zusammensetzung auf. Er wurde mit Hilfe eines Vorverdampfers dampfförmig in das Membranmodul eingespeist. Die Membranbehandlung wurde als Dampfpermeation mit einer Membran der Firma Sulzer Typ Sulzer 2201 in einer Laborversuchsanlage durchgeführt. Die Stromnummern in der folgenden Tabelle entsprechen den Bezeichnungen in Fig. 2. Komponenten mit einer Konzentration kleiner als 0,1 Massen-Teile im Gemisch sind in der Regel nicht in der Tabelle aufgeführt.

**Tabelle 1**

| Stromnummer | Stombezeichnung | Massenfluß [kg/h] | Konzentration der abzutrennenden Komponente in Massen-Teile |
|---|---|---|---|
| 1 | Frisch-Zulauf | 3,66 | 62,0 Wasser 33,7 TBA 3,4 2-Butanol 0,7 C₈-Alkohol 0,2 sonstige Stoffe |
| 3 | Retentat | 1,50 | 9,1 Wasser 80,7 TBA 8,0 2-Butanol 1,8 C₈-Alkohol 0,4 sonstige Stoffe |
| 4 | Permeat | 2,16 | 98,7 Wasser 1,1 TBA 0,1 2-Butanol 0,1 sonstige Stoffe |
| 6 | Destillat der Kolonne (5) | 1,38 | 9,9 Wasser 88,0 TBA 2,0 2-Butanol 0,1 sonstige Stoffe |
| 7 | Sumpf der Kolonne (5) | 0,12 | 0,1 TBA 75,5 2-Butanol 21,7 C₈-Alkohol 2,7 sonstige Stoffe |

Der Druck des Destillatstroms (1) betrug am Zulauf der Membran 1 bar und permeatseitig (4) an der Membran 0,055 bar. Die Kolonne (5) wurde bei 1 bar_{abs} mit einem Rücklaufverhältnis von 10 betrieben.

### 2. Beispiel

Die Abtrennung von SBA erfolgte in einer nach Fig. 3 realisierten Anlage, mit der Besonderheit, dass Kolonne (14) getrennt von der restlichen Anlage betrieben wurde. Der Kolonnendurchmesser der Kolonne (14) betrug dabei 50 mm. Es wurde eine Metall-Destillations-Packung mit 10 theoretischen Stufen installiert, der Zulauf erfolgte auf der, von oben gezählten, 5. theoretischen Stufe. Der Zulauf (13), der für die Versuche verwendet wurde, wurde aus einer großtechnischen Anlage zur Herstellung von Isobuten entnommen und wies die in Tabelle 2 angegebene Zusammensetzung auf. Das erhaltene, an Wasser abgereicherte Destillat wurde gesammelt und anschließend in der Dampfpermeation weiter an Wasser abgereichert. Für die Dampfpermeation wurde eine Membran der Firma Sulzer, Typ Sulzer 2201, in einer Laborversuchsanlage verwendet. Die Kolonne (5) entsprach dem Aufbau in Beispiel 1, mit der Besonderheit, dass das SBA als Seitenstrom (7A) oberhalb des Verdampfers aus der Packung mit Hilfe eines Flüssigkeitssammlers entnommen wurde. Zwischen dem Verdampfer und der Flüssigkeitsentnahme befand sich eine Destillationspackung, welche ca. einer theoretischen Stufe entsprach. Kolonne (8) entfiel, womit auch die Ströme 9, 10, 11 und 12 entfielen. Die Stromnummern in der folgenden Tabelle 2 beziehen sich auf die Bezeichnung der Ströme in Fig. 3. Komponenten mit einer Konzentration kleiner als 0,1 Massen-Teile im Gemisch sind in der Regel nicht in der Tabelle 2 aufgeführt.

**Tabelle 2**

| Stromnummer | Strombezeichnung | Massenfluß [kg/h] | Konzentration der abzutrennenden Komponente (Massen-Teile) |
|---|---|---|---|
| 13 | Frisch-Zulauf | 25 | 96,6 Wasser 3,0 TBA 0,3 2-Butanol 0,1 C₈-Alkohol |
| 15 | Abwasser | 23,72 | 99,9 Wasser 0,1 TBA |
| 1 (als Destillat) | Destillat | 1,28 (wurde gesammelt, für Membranversuche) | 34,6 Wasser 58,3 TBA 5,8 2-Butanol 1,0 C₈-Alkohol 0,3 sonstige Stoffe |
| 1 (als Ausgangsstoff) | Membranzulauf | Gesammeltes Produkt: 3,93 | 34,6 Wasser 58,3 TBA 5,8 2-Butanol 1,0 C₈-Alkohol 0,3 sonstige Stoffe |
| 3 | Retentat | 2,60 | 3,1 Wasser 86,3 TBA 8,6 2-Butanol 1,5 C₈-Alkohol 0,5 sonstige Stoffe |
| 4 | Permeat | 1,33 | 96,1 Wasser 3,4 TBA 0,3 2-Butanol 0,1 C₈-Alkohol |
| 6 | Destillat der Kolonne (5) | 2,34 | 3,5 Wasser 95,7 TBA 0,6 2-Butanol 0,2 sonstige Stoffe |
| 7A | Seitenstrom der Kolonne (5) | 0,19 | 0,6 TBA 98,8 2-Butanol 0,4 C₈-Alkohol 0,2 sonstige Stoffe |
| 7 | Sumpf der Kolonne (5) | 0,06 | 0,1 TBA 31,6 2-Butanol 58,8 C₈-Alkohol 9,5 sonstige Stoffe |

Der Druck des Destillatsstroms (1) betrug am Zulauf der Membran 1 bar und permeatseitig (4) an der Membran 0,055 bar. Die Kolonne (5) wurde bei 1 bar_{abs}. mit einem Rücklaufverhältnis von 5 betrieben. Die Kolonne (14) wurde bei einem Druck von 1 bar_{abs}. mit einem Rücklaufverhältnis von 2,8 betrieben.

## Patentansprüche

1. Verfahren zur Abtrennung von 2-Butanol (SBA) aus einem technischen Gemisch, das 2-Butanol, tert.-Butanol (TBA) und Wasser aufweist, wobei der Massenanteil an Wasser in dem Gemisch größer ist als die Grenzkonzentrationen der die beiden Azeotrope TBA/Wasser und SBA/Wasser verbindenden Destillationsgrenzlinie,
**dadurch gekennzeichnet,**
**dass** aus dem Gemisch mit Hilfe einer Membran so viel Wasser abgetrennt wird, dass das Retentat hinsichtlich seiner SBA/TBA/Wasser-Zusammensetzung einen Massenanteil an Wasser aufweist, der kleiner ist als die Grenzkonzentration der die beiden Azeotrope TBA/Wasser und SBA/Wasser verbindenden Destillationsgrenzlinie, und dass das Retentat destillativ in einen SBA aufweisenden Strom und einen TBA und Wasser aufweisenden Strom getrennt wird.

2. Verfahren nach Anspruch 1,
**dadurch gekennzeichnet,**
**dass** die Abtrennung von Wasser mit Hilfe einer Membran durch Pervaporation erfolgt.

3. Verfahren nach Anspruch 1,
**dadurch gekennzeichnet,**
**dass** die Abtrennung von Wasser mit Hilfe einer Membran durch Dampf-Permeation erfolgt.

4. Verfahren nach einem der Ansprüche 1 bis 3,
**dadurch gekennzeichnet,**
**dass** die Abtrennung von Wasser mit Hilfe einer Membran durch Pervaporation und Dampf-Permeation erfolgt.

5. Verfahren nach einem der Ansprüche 1 bis 4,
**dadurch gekennzeichnet,**
**dass** dem technischen Gemisch vor der Zuführung zum Membranmodul destillativ Wasser entzogen wird.

6. Verfahren nach einem der Ansprüche 1 bis 5,
**dadurch gekennzeichnet,**
**dass** eine anorganische Membran oder eine Polymermembran eingesetzt wird.

7. Verfahren nach einem der Ansprüche 1 bis 5,
**dadurch gekennzeichnet,**
**dass** eine Kombination aus anorganischer Membran bzw. anorganischem Trägermaterial und einer Polymermembran bzw. aufgebrachten Polymertrennschicht eingesetzt wird.

8. Verfahren nach einem der Ansprüche 1 bis 7,
**dadurch gekennzeichnet,**
**dass** das an Wasser abgereicherte Retentat, welches destillativ aufgearbeitet wird, einen Wassergehalt von kleiner 10 Massen-% bezogen auf das Dreikomponentensystem SBA/TBA/Wasser aufweist.

9. Verfahren nach einem der Ansprüche 1 bis 8,
**dadurch gekennzeichnet,**
**dass** das Retentat destillativ in eine 2-Butanol aufweisende Fraktion getrennt wird, die weniger als 1 Massen-% tert.-Butanol aufweist.

10. Verfahren nach einem der Ansprüche 1 bis 9,
**dadurch gekennzeichnet,**
**dass** als Gemisch ein aus einer Destillationskolonne unterhalb des Zulaufs dampfförmig oder flüssig entnommener Seitenstrom in ein Membranmodul geführt wird, dort Wasser als Permeat ausgeschleust und das Retentat in die Kolonne an gleicher oder tieferer Stelle zurückgeführt wird, wodurch erreicht wird, dass das Gemisches im unteren Teil der Kolonne eine SBA/TBA/Wasser-Zusammensetzung mit einem Massenanteil an Wasser aufweist, der kleiner ist als die Grenzkonzentration der die beiden Azeotrope TBA/Wasser und SBA/Wasser verbindenden Destillationsgrenzlinie und im Sumpf der Kolonne angereichertes SBA abtrennt wird.

## Claims

1. Process for separating 2-butanol (SBA) from an industrial mixture which comprises 2-butanol, tert-butanol (TBA) and water and in which the proportion by mass of water is greater than the limit concentrations of the distillation boundary line connecting the two azeotropes TBA/water and SBA/water, **characterized in that** water is separated off from the mixture by means of a membrane in such an amount that the retentate has, in terms of its SBA/TBA/water composition, a proportion by mass of water which is less than the limit concentration of the distillation boundary line connecting the two azeotropes TBA/water and SBA/water and **in that** the retentate is separated by distillation into a stream comprising SBA and a stream comprising TBA and water.

2. Process according to Claim 1, **characterized in that** the removal of water by means of a membrane is carried out by pervaporation.

3. Process according to Claim 1, **characterized in that** the removal of water by means of a membrane is carried out by vapor permeation.

4. Process according to any of Claims 1 to 3, **characterized in that** the removal of water by means of a membrane is carried out by pervaporation and vapor permeation.

5. Process according to any of Claims 1 to 4, **characterized in that** water is removed from the industrial mixture by distillation before it is fed to the membrane module.

6. Process according to any of Claims 1 to 5, **characterized in that** an inorganic membrane or a polymer membrane is used.

7. Process according to any of Claims 1 to 5, **characterized in that** a combination of an inorganic membrane or inorganic support material and a polymer membrane or applied polymer separation layer is used.

8. Process according to any of Claims 1 to 7, **characterized in that** the retentate which has been depleted in water and is worked up by distillation has a water content of less than 10% by mass, based on the three-component system SBA/TBA/water.

9. Process according to any of Claims 1 to 8, **characterized in that** the retentate is separated by distillation to give a 2-butanol-containing fraction containing less than 1% by mass of tert-butanol.

10. Process according to any of Claims 1 to 9, **characterized in that** a side stream taken off from a distillation column in gaseous or liquid form at a point below the feed point is fed as mixture to a membrane module, water is discharged there as permeate and the retentate is fed back into the column at the same point or a lower point, as a result of which the mixture in the lower part of the column has an SBA/TBA/water composition having a proportion by mass of water which is less than the limit concentration of the distillation boundary line connecting the two azeotropes TBA/water and SBA/water and enriched SBA is separated off at the bottom of the column.

## Revendications

1. Procédé pour la séparation de 2-butanol (SBA) à partir d'un mélange industriel qui comporte du 2-butanol, du tert-butanol (TBA) et de l'eau, la proportion en masse d'eau dans le mélange étant supérieure à la concentration limite de la frontière de distillation reliant les deux azéotropes TBA/eau et SBA/eau,
**caractérisé en ce**
**qu'**on sépare du mélange à l'aide d'une membrane une quantité d'eau telle que le rétentat, eu égard à sa composition SBA/TBA/eau, présente une teneur massique en eau qui est inférieure à la concentration limite de la frontière de distillation reliant les deux azéotropes TBA/eau et SBA/eau, et en ce qu'on fractionne par distillation le rétentat en un courant comportant du SBA et un courant comportant du TBA et de l'eau.

2. Procédé selon la revendication 1,
**caractérisé en ce que**
la séparation de l'eau s'effectue à l'aide d'une membrane par pervaporation.

3. Procédé selon la revendication 1,
**caractérisé en ce que**
la séparation de l'eau s'effectue à l'aide d'une membrane par perméation de vapeur.

4. Procédé selon l'une quelconque des revendications 1 à 3,
**caractérisé en ce que**
la séparation de l'eau s'effectue à l'aide d'une membrane par pervaporation et perméation de vapeur.

5. Procédé selon l'une quelconque des revendications 1 à 4,
**caractérisé en ce**
**qu'**avant l'introduction dans le module à membrane on élimine l'eau par distillation du mélange industriel.

6. Procédé selon l'une quelconque des revendications 1 à 5,
**caractérisé en ce**
**qu'**on utilise une membrane inorganique ou une membrane polymère.

7. Procédé selon l'une quelconque des revendications 1 à 5,
**caractérisé en ce**
**qu'**on utilise une combinaison de membrane inorganique ou de matière de support inorganique et d'une membrane polymère ou d'une couche de séparation polymère appliquée.

8. Procédé selon l'une quelconque des revendications 1 à 7,
**caractérisé en ce que**
le rétentat appauvri en eau, qui est traité par distillation, présente une teneur en eau de moins de 10 % en masse, par rapport au système tricomposant SBA/TBA/eau.

9. Procédé selon l'une quelconque des revendications 1 à 8,
**caractérisé en ce**
**qu'**on fractionne par distillation le rétentat en une fraction comportant du 2-butanol, qui comporte moins de 1 % en masse de tert-butanol.

10. Procédé selon l'une quelconque des revendications 1 à 9,
**caractérisé en ce**
**qu'**on envoie dans un module à membrane en tant que mélange un courant latéral prélevé à l'état liquide ou de vapeur d'une colonne de distillation au-dessous du conduit d'alimentation, l'eau y est évacuée en tant que perméat et le rétentat est recyclé au niveau du même point ou en un point inférieur dans la colonne, de sorte qu'on parvient à ce que le mélange dans la partie inférieure de la colonne présente une composition SBA/TBA/eau ayant une teneur massique en eau qui est inférieure à la concentration limite de la frontière de distillation reliant les deux azéotropes TBA/eau et SBA/eau, et dans le pied de la colonne on sépare du SBA enrichi.
